(19) Europäisches Patentamt
European Patent Office
Office européen des brevets

(11) Veröffentlichungsnummer: **0 044 442**
**B1**

(12) # EUROPÄISCHE PATENTSCHRIFT

(45) Veröffentlichungstag der Patentschrift: **15.02.84**

(21) Anmeldenummer: **81105025.1**

(22) Anmeldetag: **29.06.81**

(51) Int. Cl.³: **C 07 D 277/46**, C 07 D 417/02, C 07 D 417/04, C 07 D 417/10, A 61 K 31/425 // (C07D417/04, 277/46, 213/38)

(54) **N-(4-Phenyl-2-thiazolyl)-oxamidsäure-2-ethoxyethylester, Verfahren zu seiner Herstellung und diesen enthaltende therapeutische Mittel.**

(30) Priorität: **19.07.80 DE 3027527**

(43) Veröffentlichungstag der Anmeldung: **27.01.82 Patentblatt 82/4**

(45) Bekanntmachung des Hinweises auf die Patenterteilung: **15.02.84 Patentblatt 84/7**

(84) Benannte Vertragsstaaten: **AT BE CH DE FR GB IT LI LU NL SE**

(56) Entgegenhaltungen:
**EP - A - 0 006 368**
**CH - A - 460 017**
**DE - A - 2 132 431**
**DE - A - 2 144 683**
**DE - A - 2 828 091**

(73) Patentinhaber: **BASF Aktiengesellschaft, Carl-Bosch-Strasse 38, D-6700 Ludwigshafen (DE)**

(72) Erfinder: **Eilingsfeld, Heinz, Dr., Pierstrasse 9 A, D-6710 Frankenthal (DE)**
Erfinder: **Neumann, Peter, Dr., Franz-Schubert-Strasse 1, D-6908 Wiesloch (DE)**
Erfinder: **Seybold, Guenther, Dr., Friedrich-Ebert-Strasse 14, D-6701 Neuhofen (DE)**
Erfinder: **Lenke, Dieter, Dr., Kekuleplatz 1, D-6700 Ludwigshafen (DE)**
Erfinder: **Friedrich, Ludwig, Dr., Nibelungenstrasse 8A, D-6831 Bruehl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# N-(4-Phenyl-2-thiazolyl)-oxamidsäure-2-ethoxyethylester, Verfahren zu seiner Herstellung und diesen enthaltende therapeutische Mittel

Die Erfindung betrifft N-(4-Phenyl-2-thiazolyl)-oxamidsäure-2-ethoxyethylester und diesen enthaltende therapeutische Mittel, die bei der Behandlung allergischer Erkrankungen verwendet werden können.

Es sind eine Reihe von Derivaten der Oxamidsäure und ihrer Ester bekannt, beispielsweise aus der DE-OS 24 13 966 Aryl- und Hetarylderivate, Thiazolderivate aus der DE-OS 28 28 091 und der veröffentlichten europäischen Patentanmeldung 0006368 und Benzothiazolderivate aus der DE-OS 27 51 441 und 26 56 486, für die eine Verwendung bei der Bekämpfung oder Unterdrückung von allergischen Reaktionen beschrieben wird. Ihre Wirkungen befriedigen jedoch nicht immer.

Es wurde nun gefunden, dass N-(4-Phenyl-2-thiazolyl)-oxamidsäure-2-ethoxyethylester (Formel I)

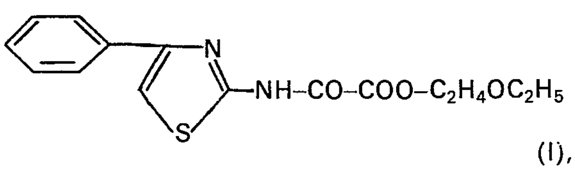

(I),

wertvolle pharmakologische Eigenschaften, insbesondere als Antiallergikum, aufweist.

Die erfindungsgemässe Verbindung der Formel I wird hergestellt, indem man das 2-Amino-4-phenyl-thiazol (II)

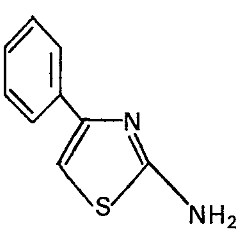

II

mit einem Oxalsäureesterhalogenid der Formel III

$$C_2H_5OC_2H_4-O-\underset{\underset{O}{\|}}{C}-\underset{\underset{O}{\|}}{C}-Hal \quad III,$$

in der Hal ein Halogenatom, bevorzugt ein Chloratom, bedeutet, zweckmässig in einem Lösungsmittel und in Anwesenheit eines Säureakzeptors in an sich bekannter Weise umsetzt.

Geeignete Lösungsmittel sind beispielsweise Methylenchlorid, Chloroform, Tetrachlorkohlenstoff, 1,2-Dichloräthan, 1,1,1-Trichloräthan, Essigsäureethylester, Dimethylformamid, Dimethylacetamid, N-Methylpyrrolidon, Tetrahydrothiophendioxid, Hegamethylphosphorsäuretriamid, Tetrahydrofuran, Dioxan oder Mischungen derselben. Beispiele für Säureakzeptoren sind Triethylamin, Tributylamin, Pyridin. Die Reaktion zwischen den Aminothiazolen II und den Oxalylhalogeniden III wird vorzugsweise bei relativ niederen Temperaturen durchgeführt, wobei Temperaturen zwischen 0 °C und 30 °C üblich sind. An Stelle der freien Aminothiazole kann man die Hydrochloride verwenden, wenn man ein zusätzliches Äquivalent Base einsetzt.

Der Oxamidsäureester der Formel I lässt sich ausserdem nach üblichen, beispielsweise in der schweizerischen Patentschrift 512 257 beschriebenen Verfahren durch Umesterung der entsprechenden Oxamidsäureniedrigalkylester mit 2-Ethoxyethanol gewinnen.

Der erfindungsgemässe Oxamidsäureester kann auch aus der entsprechenden freien Oxamidsäure nach Überführung in das Oxamidsäurechlorid durch Veresterung mit 2-Ethoxyethanol in Gegenwart eines säurebindenden Mittels, wie Triethylamin, in an sich üblicher Weise hergestellt werden.

Die Veresterung der Oxamidsäure kann auch in Gegenwart eines sauren Katalysators oder eines Carbodiimids, wie es in Chem. Ber. 100, 16 (1967) bzw. in Acta Chem. Scand. B 33, 410 (1979) beschrieben ist, durchgeführt werden. Die Veresterung der freien Oxamidsäure kann ausserdem mittels eines Dimethylformamid-acetals nach der Imidazolid-Methode [Chem. Ber. 95, 1284 (1962)] oder nach dem in Bull. Chem. Soc. Japan 50, 1863 (1977 beschriebenen Verfahren erfolgen.

Das als Ausgangsstoff verwendete 2-Amino-4-phenylthiazol (II) ist bekannt.

Die erfindungsgemässe Verbindung weist antiallergische Eigenschaften, aufgrund deren sie als wertvolle Heilmittel bei der Behandlung von allergischen Erkrankungen des Respirations-, des Gastro-Intestinaltraktes und der Haut, beispielsweise allergischen Asthmas, allergischen Rhinitis oder Nahrungsmittel-Allergien, verwendet werden kann, auf. Gegenüber dem bekannten Antiallergikum Cromolyne zeichnet sich die erfindungsgemässe Verbindung im Tierexperiment bei der passiven cutanen Anaphylaxie der Ratte dadurch aus, dass sie oral wirksam ist und zusätzlich über eine wesentlich längere Wirkdauer verfügt.

Die antiallergische Wirkung wurde an Ratten getestet. Zur Prüfung wurde das Modell der passiven cutanen Anaphylaxie (PCA) verwendet.

Narkotisierte männliche Ratten (100 bis 140 g) werden durch intradermale Injektion (rasierte Rückenhaut) von 0,1 ml eines Ovalbumin-Antiserums sensibilisiert. Nach einer Sensibilisierungsperiode von ca. 48 Stunden erfolgt die Behandlung (intraperitoneale bzw. orale Applikation) mit unterschiedlichen Dosierungen (10 Tiere/Dosis) der Prüfsubstanz. 15 bzw. 20 Minuten nach der Behandlung wird eine Antigen/Evansblau-Mischung (10 mg/kg Ovalbumin in 2%iger Evansblau-Lösung) den Versuchstieren intravenös injiziert. 30 Minuten später werden die Tiere getötet, die Rückenhaut abgezogen und auf der inneren Oberfläche der Durchmesser der kreisförmigen Blaufärbung gemessen. Die Grösse der Farbflecke unbehandelter Kontrolltiere ist

standardisierbar. Antiallergisch wirksame Substanzen verkleinern dosisabhängig den Durchmesser der Farbflecke. Als ED 50% wird die Dosis angegeben die im Vergleich zu medikamentös unbehandelten Kontrolltieren den Durchmesser der Farbflecke um 50% herabsetzt.

Ausser der antiallergischen Wirkung wurde die akute Toxizität an Gruppen von je 2 NMRI-Mäusen, Gewicht 20 bis 26 g, bei intraperitonealer Applikation und an je 2 Sprague-Dawley-Ratten, Gewicht 120 bis 150 g, bei oraler Applikation ermittelt. Die Bestimmung der LD 50 erfolgte an Gruppen von je 10 NMRI-Mäusen, Gewicht 20 bis 26 g, nach intraperitonealer Applikation. Die Nachbeobachtungszeit betrug 14 Tage.

Die erfindungsgemässe Verbindung ist antiallergisch hochwirksam. Aus Tabelle 1 geht hervor, dass diese Substanz nach oraler Gabe 227 mal wirksamer ist als die Vergleichsverbindung $V_1$ (N-(4-Phenyl-2-thiazolyl) -oxamidsäure-2-ethylester, Beispiel 1 der europäischen Patentveröffentlichung 000 6368). Die Handelssubstanz Cromolyne ist in dieser Versuchsanordnung bis 100 mg/kg p.o. unwirksam.

Tabelle 1:
Antiallergische Wirkung und akute Toxizität

| Substanz | Hemmung der PCA. ED 50% mg/kg | Ratte per os R.W. | LD 50 mg/kg Maus i.p. | Ratte p.o. |
|---|---|---|---|---|
| I | 0,0382 | 227 | 956 | >2 150 |
| $V_1$ | 8,67 | 1 | ca. 681 | >2 150 |
| Cromolyne | 100 | – | – | >2 150 |

[1]) Relative Wirksamkeit

Aufgrund der besseren Wirksamkeit ergibt sich ein wesentlich grösserer Abstand zwischen der toxischen und der wirksamen Dosis.

Für die therapeutische Anwendung werden Einzeldosen von 0,1 bis 100 mg verwendet.

Gegenstand der vorliegenden Erfindung sind demnach auch therapeutische Mittel oder Zubereitungen, die neben üblichen Träger- und Verdünnungsmitteln die Verbindung der Formel I als Wirkstoff enthalten, sowie die Verwendung der neuen Verbindung bei der Behandlung von allergischen Erkrankungen.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Suspensionen oder Depotformen. Weiterhin kommen Inhalate und parenterale Zubereitungen, wie Injektionslösungen, in Betracht.

Die galenischen Applikationsformen, fest oder flüssig, werden in üblicher Weise hergestellt. Der Wirkstoff kann dabei mit den gebräuchlichen galenischen Hilfsmitteln, wie Talkum, Gummi arabicum, Saccharose, Lactose, Getreide- oder Maisstärke, Kartoffelmehl, Magnesiumstearat, Alginaten, Gummi tragacanth, Carraghenate, Polyvinylalkohol, Polyvinylpyrrolidon, wässrigen oder nicht wässrigen Trägern, Netzmitteln, Dispergiermitteln, Emulgatoren und/oder Konservierungsmitteln verarbeitet werden (vgl. L.G. Goodman, A. Gilman, The Pharmacological Basis of Therapeutics).

Das nachstehende Beispiel soll die Erfindung näher beschrieben.

Beispiel

N-(4-Phenyl-2-thiazolyl)-oxamidsäure-2-ethoxyethylester

6,2 g N-(4-Phenyl-2-aminothiazolyl)-oxamidsäure (hergestellt aus dem Ethylester durch Verseifen mit wässrigem Kaliumacetat und Freisetzen der Säure mit verdünnter Salzsäure) werden in 25 ml trockenem Toluol mit 20 ml Oxalylchlorid 2 Stunden auf 60 °C erwärmt. Nach Abdestillieren des überschüssigen Oxalylchlorids unter vermindertem Druck tropft man eine Lösung von 5,6 g Ethylenglykolmonoethylether, 3,5 ml Triethylamin und 20 ml Toluol zu. Man rührt 3 Stunden bei Raumtemperatur nach und lässt über Nacht stehen. Der Niederschlag wird abgesaugt, mit Methanol und Wasser gewaschen und aus Ethanol umkristallisiert. Man erhält 3,3 g der Verbindung der Formel I vom Schmp. 109 bis 110 °C.

$C_{15}H_{16}O_4N_2S$ (320)

| | C | H | O | N | S |
|---|---|---|---|---|---|
| Ber. | 56,24 | 5,03 | 19,98 | 8,74 | 10,01 |
| Gef. | 56,3 | 5,0 | 19,6 | 8,9 | 10,1 |

Beispiele für pharmazeutische Zubereitungen

Tabletten

| | | |
|---|---|---|
| a) | Wirkstoff I | 0,100 g |
| | Stearinsäure | 0,010 g |
| | Traubenzucker | 1,850 g |
| | | 2,000 g |
| b) | Wirkstoff I | 0,020 g |
| | Stearinsäure | 0,020 g |
| | Traubenzucker | 1,960 g |
| | | 2,000 g |

Die Bestandteile werden in üblicher Weise zu Tabletten der vorstehend angegebenen Zusammensetzung verarbeitet.

Inhalationsaerosol

| | |
|---|---|
| Wirkstoff I | 1,00 Teile |
| Sojalecithin | 0,20 Teile |
| Treibgasmischung (Frigen 11, 12 und 114) ad | 100,00 Teile |

Die Zubereitung wird vorzugsweise in Aerosolbehälter mit Dosierventil abgefüllt. Der einzelne Hub wird so bemessen, dass eine Dosis von 5 bis 20 mg Wirkstoff angegeben wird.

Ampullen (Injektionslösungen)

| | |
|---|---|
| Wirkstoff I | 50,0 Gew.-Teile |
| Natriumpyrosulfit | 1,0 Gew.-Teil |
| Dinatriumsalz der Äthylendiamintetraessigsäure | 0,5 Gew.-Teile |
| Natriumchlorid | 8,5 Gew.-Teile |
| doppelt destilliertes Wasser ad | 1000,0 Gew.-Teile |

Der Wirkstoff und die Hilfsstoffe werden in einer ausreichenden Menge Wasser gelöst und mit der notwendigen Menge Wasser auf die gewünschte Konzentration gebracht. Die Lösung wird filtriert und unter aseptischen Bedingungen in 1 ml-Ampullen abgefüllt. Zuletzt werden die Ampullen sterilisiert und verschlossen.

**Patentansprüche**

1. N-(4-Phenyl-2-thiazolyl)-oxamidsäure-2-ethoxyethylester.

2. Therapeutische Mittel, enthaltend die Verbindung nach Anspruch 1 als Wirkstoff neben üblichen Trägerstoffen und Verdünnungsmitteln.

**Revendications**

1. 2-éthoxyéthylester de l'acide N-(4-phényl-2-thiazolyl)-oxamidique.

2. Agent thérapeutique contenant le composé selon la revendication 1 comme principe actif à côté de matières supports usuelles et de diluants.

**Claims**

1. 2-Ethoxyethyl N-(phenyl-thiazol-2-yl)-oxamate.

2. A therapeutic agent which contains the compound as claimed in claim 1 as the active compound, in addition to conventional carriers and diluents.